# EUROPEAN PATENT SPECIFICATION

(11) **EP 2 802 323 B1**
(45) Date of publication and mention of the grant of the patent: **10.08.2016**
(21) Application number: 13705838.4
(22) Date of filing: 11.01.2013
(51) Int. Cl.: A61K 31/4025, A61P 17/16, A61K 33/24, A61N 5/10, C07D 405/04

(54) **PYRROLIDINE-SUBSTITUTED FLAVONE DERIVATIVES FOR PREVENTION OR TREATMENT OF ORAL MUCOSITIS**
PYRROLIDINE-SUBSTITUIERTE FLAVONDERIVATE ZUR PRÄVENTION ODER BEHANDLUNG ORALER MUCOSITIS
DÉRIVÉS DE FLAVONE SUBSTITUÉS PAR PYRROLIDINE POUR LE TRAITEMENT DE LA MUCOSITE ORALE

(30) Priority: 13.01.2012 US 201261586428 P
(43) Date of publication of application: 19.11.2014
(73) Proprietor: Piramal Enterprises Limited, Mumbai 400 013 (IN)
(72) Inventor: SHARMA, Somesh, Los Altos, CA 94022 (US); HATFIELD, Alan, Mumbai 400063 (IN); ACHARYA, Shivani, Navi Mumbai 410210 (IN)
(74) Representative: Richards, Michèle E.
(86) International application number: PCT/IB2013/050263
(87) International publication number: WO 2013/105056

(56) References cited:
- WO-A1-2007/148158
- WO-A1-2010/128443
- LEILA GHASSEMI, EBRAHIM ZABIHI, RABI MAHDAVI, MARYAM SEYEDMAJIDI, SADAT AKRAM, MINA MOTALLEBNEJAD: "The effect of ethanolic extract of propolis on radiation-induced mucositis in rats", SAUDI MEDICAL JOURNAL, vol. 31, no. 6, 2010, pages 622-626, XP002698398,

## Description

### FIELD OF THE INVENTION

The present invention relates to pyrrolidine substituted with flavone derivatives, represented by the compounds of Formula (I) (as described herein) or pharmaceutically acceptable salts, solvates, stereoisomers or diastereoisomers thereof or pharmaceutical compositions containing the compounds of Formula (I) for use in the prevention or treatment of oral mucositis.

### BACKGROUND OF THE INVENTION

Oral mucositis is the most common debilitating complication of cancer treatment (e.g. chemotherapy, radiation therapy or a combination thereof), including bone marrow transplantation or stem cell transplantation. Oral mucositis causes considerable pain and morbidity and is often the dose-limiting toxicity of cancer treatment. Oral mucositis may also limit the patient's ability to tolerate either chemotherapy or radiotherapy or both. Oral mucositis is associated with odynophagia (painful swallowing), dysphagia (difficulty in swallowing) including feeding tube dependency and dysgeusia (distortion and/or decrease of the sense of taste). This suppresses the appetite and further adds to diminishing adequate nutrition and caloric intake. Reduction of caloric intake can lead to weight loss, malnutrition, loss in muscle mass strength and other complications including decrease in immunity. Oral mucositis is also associated with dehydration, and potential life threatening aspiration. Patients with high grade mucositis (grade 3 or 4) and reduced immunity are prone to opportunistic mouth infections. The damaged oral mucosa serves as portals of entry for endogenous oral microorganisms and therefore is a significant risk factor for life-threatening systemic infections as well.

Oral mucositis frequently occur in patients with squamous cell carcinoma of the head and neck (SCCHN) who are treated with radiation therapy directed at the oral and pharyngeal regions. According to Trotti et al. (Radiother. Oncol., 2003; 66, 253-262) the overall incidence of mucositis in this patient population of 6,000 patients with SCCHN was 80%, with 39% of cases being grade 3/4, which limited or prevented alimentation. The said patient population received radiation therapy with or without chemotherapy. In patients who received only chemotherapy, the incidence of mucositis was 22%. Between 50% and 100% of patients undergoing stem cell transplantation (SCT) experience mucositis as a result of high-dose chemotherapy or total-body irradiation (TBI). Sonis et al. (J Clin Oncol 2001;19:2201-2205) reported that a higher oral mucositis rating correlated with an increased risk of significant infection, an increased number of days in the hospital, a greater use of opioids and total parenteral nutrition (TPN), higher healthcare costs, and an elevated 100-day mortality rate.

Oral mucositis is not only a common consequence of radiation therapy or combination of chemotherapy and radiation therapy, but is also caused to patients undergoing bone marrow transplantation (BMT). BMT has been found to be successful in the treatment of leukemia, lymphoma and some solid mass tumours. Prior to a BMT, intensive chemotherapy and total body irradiation (for allogenic BMT patients) is administered to the patient in an effort to destroy all cancer cells. At this stage the oral mucositis is caused to BMT patients.

Since the healthcare cost associated with oral mucositis and its treatment can be substantial, prompt and accurate diagnosis and initiation of prophylaxis and treatment of oral mucositis are essential.

The management of oral mucositis currently focuses on maintaining oral hygiene and pain control. Good oral hygiene promotes patient comfort and helps to prevent superimposed infection. Adequate analgesia is essential both to control pain and to ensure maximum possible oral nutritional intake. Paracetamol in conjunction with stronger analgesics such as codeine, dihydrocodiene or strong opiates such as morphine can be used for pain relief. MASCC (Multinational Association of Supportive Care in Cancer) guidelines recommend the use of Benzydamine hydrochloride mouthwash for the prevention of oral mucositis in patients with head and neck cancer receiving moderate-dose radiation therapy or chemotherapy or combination thereof. Antibiotic and antifungal medication can be used for the treatment of mouth infections post cancer therapy.

A number of targeted therapies have recently been evaluated for prevention and/or treatment of oral mucositis, including palifermin, amifostine, glutamine, cytokines growth factors, and other antioxidants.

Palifermin, a human recombinant keratinocyte growth factor produced by *E.coli*, is approved for the treatment of severe oral mucositis. It is known to reduce the incidence and duration of severe oral mucositis by binding to epithelial cell-surface receptors and stimulating epithelial cell proliferation, differentiation, and upregulation of cytoprotective mechanisms. However, palifermin is associated with skin toxicities such as rash; erythema; edema; and pruritus; oral toxicities such as dysesthesia; tongue discoloration; tongue thickening and alteration of taste, and pain arthralgias (www.accessdata.fda.gov: "Kepivance® (palifermin)- For injection, for intravenous use- Label Approved by the USFDA-Supplement number 0018).

Amifostine, a cytoprotective prodrug has been extensively studied for its role in prevention of oral mucositis induced by radiation therapy or chemotherapy. It showed statistically significant role in the reduction of severity of oral mucositis. However, amifostine therapy is associated with several drawbacks such as hypocalcemia, diarrhea, nausea, vomiting, sneezing, somnolence, hiccoughs, more serious side-effects such as hypotension (found in 62% of patients), erythema multiforme, Stevens-Johnson syndrome and toxic epidermal necrolysis, immune hypersensitivity syndrome, erythroderma, anaphylaxis, and loss of consciousness (rare). In addition, amifostine requires daily intravenous infusions. Due to the inconsistent results, amifostine was not approved by US FDA for oral mucositis.

Glutamine, a nonessential amino acid reduces mucosal injury by reducing the production of pro-inflammatory cytokines and cytokines related apoptosis. Many malignancies are characterized by decreased glutamine levels, which can be further exacerbated by cell damage caused by cancer therapy. Glutamine supplementation can reverse this effect and may help to protect mucosal tissues from damage by radiation therapy or chemotherapy and thus accelerate recovery. But many trials using glutamine as oral or systemic supplement and as mouth washes have shown inconsistent results. Due to which MASCC and ISOO (International Society of Oral Oncology) did not recommend its routine use.

Granulocyte colony-stimulating factor (G-CSF) and granulocyte-macrophage colony stimulating factor (GM-CSF) have shown protective effects against radiation induced mucositis. The systemic and local use as topical mouth wash of G-CSF and GM-CSF, respectively, has been evaluated in different trials for the prevention and treatment of oral mucositis. But the results of these trials favor the systemic intervention group however no preventive effect was found for the topical administration group. In view of inconsistent results MASCC/ISOO guidelines did not recommend the routine use of GM-CSF and G-CSF in any form for the prevention or treatment of oral mucositis.

Despite the availability of certain therapeutic options for the treatment of oral mucositis which is debilitating complication of cancer treatment, effective prevention or treatment of this severe side effect of cancer treatment still constitutes a challenging job for medical practitioners. Therefore, newer treatment options for oral mucositis are needed.

There are a large numbers of cancer patients particularly those undergoing radiation therapy for head and neck cancers, who often receive multiple cycles of radiation and hence, suffer from radiation related toxicity. Mucositis is arguably the most significant radiation induced toxicity associated with head and neck cancer therapy. Despite the availability of certain therapeutic options for the treatment of mucositis, an effective prevention or treatment of this severe side effect still constitutes a challenging job for medical practitioners. Therefore, newer treatment options are needed.

The inventors of the present invention have now surprisingly found out that pyrrolidine substituted with flavone according to the claims can be used for the prevention or treatment of oral mucositis.

The invention described herein provides pyrrolidine substituted with flavone derivatives represented by Formula (I) (as described herein) for the prevention and treatment of oral mucositis caused by cancer therapy.

### SUMMARY OF THE INVENTION

According to one aspect of the invention, there is provided a compound of Formula (I) (as described herein), a pharmaceutically acceptable salt, a solvate, or a stereoisomer or a diastereoisomer thereof for use in the prevention, treatment or reduction in severity of oral mucositis in a subject undergoing cancer therapy.

According to another aspect of the invention, there is provided a compound for use in a method for the prevention, treatment or reduction in severity of oral mucositis in a subject undergoing cancer therapy, comprising administering to the subject a therapeutically effective amount of a compound of Formula (I), or a pharmaceutically acceptable salt, a solvate, a stereoisomer or a diastereoisomer thereof.

According to another aspect of the invention, there is provided a compound for use in a method for prevention, treatment or reduction in the severity of oral mucositis in a subject undergoing cancer therapy, comprising administering the compound of Formula (I) to the subject, prior to, concurrently with or after the cancer therapy.

According to another aspect of the invention, there is provided a compound of Formula (I), a pharmaceutically acceptable salt, a solvate, or a stereoisomer or a diastereoisomer thereof for use in the prevention, treatment or reduction in severity of oral mucositis in a subject undergoing cancer therapy.

According to another aspect of the invention there is provided a pharmaceutical composition comprising a therapeutically effective amount of a compound of Formula (I) a pharmaceutically acceptable salt, a solvate, a stereoisomer or a diastereoisomer thereof and a pharmaceutically acceptable carrier for use in the treatment of oral mucositis in a subject undergoing cancer therapy.

Other aspects and further scope of applicability of the present invention will become apparent from the detailed description to follow.

Any subject-matter which is not encompassed by the scope of the claims does not form part of the presently claimed invention.

### DETAILED DESCRIPTION OF THE INVENTION

The general terms used hereinbefore and hereinafter preferably have within the context of this disclosure the following meanings, unless otherwise indicated. Thus, the definitions of the general terms as used in the context of the present invention are provided herein below:

The singular forms "a," "an," and "the" include plural reference unless the context clearly dictates otherwise.

It will be understood that "substitution" or "substituted with" includes the implicit proviso that such substitution is in accordance with permitted valence of the substituted atom and the substituent, as well as represents a stable compound, which does not readily undergo transformation such as rearrangement, cyclization, elimination, etc.

The term "C₁-C₄- alkyl" refers to the radical of saturated aliphatic groups, including straight or branched-chain containing from 1 to 4 carbon atoms. Examples of alkyl groups include methyl, ethyl, propyl, butyl, isopropyl, isobutyl, *sec*-butyl and *tert*-butyl. The term "C₁-C₄ - alkoxy" refers to an alkyl group as defined above attached via oxygen linkage to the rest of the molecule. Examples of alkoxy include methoxy, ethoxy, propoxy, butoxy and tert-butoxy.

The term "halogen" refers to fluorine, chlorine, bromine and iodine.

The term "hydroxy" or "hydroxyl" as used herein, refers to -OH group.

The term "therapeutically effective amount", as used herein refers to the amount of a compound represented by Formula (I), a pharmaceutically acceptable salt, a solvate, a stereoisomer or a diastereoisomer thereof, that, when administered to a subject in need of such treatment, is sufficient to provide therapeutic benefit, including, the prevention, treatment or amelioration of oral mucositis; such that any toxic or detrimental effects of the composition of compound of Formula (I) is outweighed by its therapeutically beneficial effects. The precise desired therapeutic effect will vary according to the disease state, the formulation to be administered, age, sex, and weight of the individual and a variety of other factors that are appreciated by those of ordinary skill in the art.

The term "subject" as used herein, refers to an animal, preferably a mammal, most preferably a human, being treated. More particularly, a human suffering from solid and/or haematological cancer. The term "mammal" as used herein is intended to encompass humans, as well as non-human mammals which are susceptible to oral mucositis. Non-human mammals include domestic animals, such as cows, pigs, horses, dogs, cats, rabbits, rats and mice, and non-domestic animals.

The term "prevention" as used herein refers to the prophylactic effect. The term means preventing the complete or partial occurrence of oral mucositis.

The term "treat" or "treatment" or "treating" as used herein, includes, curative, alleviative or prophylactic effects. The term includes (i) reduction in the progression of oral mucositis or (ii) reduction in the severity of oral mucositis or (iii) reduction in the frequency of development of oral mucositis or (iv) reduction in the duration of oral mucositis or (v) amelioration and/or relief of one or more signs or symptoms associated with oral mucositis.

The term "oral mucositis" as used herein, refers to inflammation of mucosal cells of the oral cavity. Oral mucositis is characterized by pain, redness, inflammation, ulceration, or combinations thereof, which results from cancer therapy such as radiation therapy, chemotherapy or both.

The term "head and neck cancer" as used herein, refers to the cancer originating in the head and neck area, comprising nasal cavity, sinuses, lips, mouth, salivary glands, throat, and larynx.

The term "reduction in the severity of oral mucositis" as used herein refers to reduction in grade 3 and above of oral mucositis, in comparison to the control group. Grade 3 and above are as defined in Common Terminology Criteria for Adverse Events (version 3) laid down by the National Cancer Institute.

As used herein the term "cancer therapy" encompasses within its scope radiation therapy, chemotherapy, hematopoietic stem cell transplantation, bone marrow transplantation or a combination thereof.

The term "subject undergoing cancer therapy" as used herein refers to an animal, preferably a mammal, most preferably a human, who is suffering from solid or haematological cancer, is exposed to or is going to be exposed to cancer therapy, for the treatment of the solid or haematological cancer.

The term "about" as used herein refers to the deviation in the numerical values by ± 10%.

As used herein the term "pharmaceutically acceptable" is meant that the carrier, diluent, excipients, and/or salt must be compatible with the other ingredients of the formulation, and not deleterious to the recipient thereof. "Pharmaceutically acceptable" also means that the compositions or dosage forms are within the scope of sound medical judgment, suitable for use for an animal or human without excessive toxicity, irritation, allergic response, or other problem or complication, commensurate with a reasonable benefit/risk ratio.

The present invention furthermore includes all solvates of the compounds of the Formula (I), for example hydrates, and the solvates formed with other solvents of crystallization, such as alcohols, ethers, ethyl acetate, dioxane, dimethylformamide or a lower alkyl ketone, such as acetone, or mixtures thereof. Certain compounds of the present invention can exist in unsolvated forms as well as solvated forms, including hydrated forms. Certain compounds of the present invention may exist in multiple crystalline or amorphous forms. In general, all physical forms are equivalent for the uses contemplated by the present invention and are intended to be within the scope of the present invention.

According to one aspect of the present invention, there is provided a compound of Formula (I), 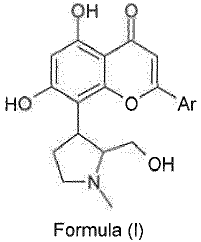 wherein Ar is a phenyl group, which is substituted by 1, 2, or 3 identical or different substituents selected from chlorine, bromine, fluorine, iodine, C₁-C₄-alkyl and trifluoromethyl; or a pharmaceutically acceptable salt, a solvate, a stereoisomer or a diastereoisomer thereof, for use in the prevention, treatment or reduction in severity of oral mucositis in a subject undergoing cancer therapy.

According to one embodiment of the present invention, there is provided a compound of Formula (I), a pharmaceutically acceptable salt, a solvate, a stereoisomer or a diastereoisomer thereof, wherein Ar is a phenyl group substituted by 1, 2, or 3 identical or different halogens selected from chlorine, bromine, fluorine and iodine, for use in the prevention, treatment or reduction in severity of oral mucositis in a subject undergoing cancer therapy.

According to another embodiment of the present invention, there is provided a compound of Formula (I), a pharmaceutically acceptable salt, a solvate, a stereoisomer or a diastereoisomer thereof, wherein Ar is a phenyl group substituted by chlorine, for use in the prevention, treatment or reduction in severity of oral mucositis in a subject undergoing cancer therapy.

According to another embodiment of the present invention there is provided a compound of Formula (I), a pharmaceutically acceptable salt, a solvate, a stereoisomer or a diastereoisomer thereof, wherein Ar is phenyl group substituted with two different substituents namely chlorine and trifluromethyl, for use in prevention, treatment or reduction in severity of oral mucositis in a subject undergoing cancer therapy.

It will be appreciated by those skilled in the art that the compounds of Formula (I) contain at least two chiral centres and hence, exists in the form of two different optical isomers (i.e., (+) or (-) enantiomers), two different geometric isomers (cis and trans) and 4 different diasteroisomers. All such enantiomers, geometric isomers, diasteroisomers and mixtures thereof including racemic mixtures are included within the scope of the invention. The enantiomers of the compound of Formula (I) can be obtained by methods disclosed in PCT Application Publication Nos. WO2004004632, WO2007148158 and WO2008007169 or the enantiomers of the compound of Formula (I) can also be obtained by methods well known in the art, such as chiral HPLC and enzymatic resolution. Alternatively, the enantiomers of the compounds of Formula (I) can be synthesized by using optically active starting materials.

The manufacture of the compounds of Formula (I), which may be in the form of pharmaceutically acceptable salts, and the manufacture of pharmaceutical composition suitable for oral, topical and/or parenteral administration containing the above compounds are generally disclosed in US Application Publication No. US20070015802.

As indicated herein above the compound of Formula (I) may be used in the form of their salts. Preferred salt of compounds of Formula (I) include acetates, alginates, ascorbates, aspartates, benzoates, benzenesulfonates, bisulfates, borates, cinnamates, citrates, ethanesulfonates, fumarates, glucuronates, glutamates, glycolates, hydrochlorides, hydrobromides, hydrofluorides, ketoglutarates, lactates, maleates, malonates, mesylate, nitrates, oxalates, palmoates, perchlorates, phosphates, picrates, salicylates, succinates, sulfamate, sulfates, tartrates, tosylate, trifluoroacetic acid salt and other acid addition salts known to the person skilled in the art.

According to another aspect of the invention there is provided a (+)-trans isomer of the compound of Formula (I), as indicated in Formula (IA) below, 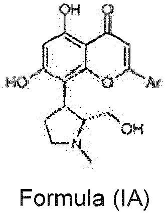 wherein Ar is a phenyl group, which is substituted by 1, 2, or 3 identical or different substituents selected from chlorine, bromine, fluorine, iodine, C₁-C₄-alkyl and trifluoromethyl, or a pharmaceutically acceptable salt or a solvate thereof, for use in the prevention, treatment or reduction in severity of oral mucositis in a subject undergoing cancer therapy.

Accordingly, in another aspect of the invention, the compound of Formula (IA) for use in the prevention, treatment or reduction in severity of oral mucositis in a subject undergoing cancer therapy is selected from (+)-*trans*-2-(2-Chloro-phenyl(-5,7-dihydroxy-8-(2-hydroxy-methly-pyrrolidin-3-yl)-chromen-4-one hydrochloride (referred to herein as compound A) or (+)-trans-3-[2[(2-Chloro-4-trifluoromethyl-phenyl)-5,7-dihydroxy-8-(2-hydroxymethyl-1-methyl-pyrrolidin-3-yl)-chromen-4-one hydrocholoride (referred to herein as compound B).

Compounds A and B are disclosed in PCT Application Publication WO2007148158 and specifically as Example 10 and Example 44, respectively.

In an embodiment, the compound of Formula (IA) for use in the prevention, treatment or reduction in severity of oral mucositis in a subject undergoing cancer therapy is (+)-*trans*-2-(2-Chloro-phenyl(-5,7-dihydroxy-8-(2-hydroxy-methly-pyrrolidin-3-yl)-chromen-4-one hydrochloride (compound A).

In another embodiment, the compound of Formula (IA) for use in the prevention, treatment or reduction in severity of oral mucositis in a subject undergoing cancer therapy is (+)-*trans*-3-[2[(2-Chloro-4-trifluoromethyl-phenyl)-5,7-dihydroxy-8-(2-hydroxymethyl-1-methyl-pyrrolidin-3-yl)-chromen-4-one hydrochloride (compound B).

Also disclosed is a method for the prevention, treatment or reduction in severity of oral mucositis in a subject undergoing cancer therapy, comprising administering to the subject a therapeutically effective amount of a compound of Formula (I), a pharmaceutically acceptable salt, a solvate, a stereoisomer or a diastereoisomer thereof.

Also disclosed is a method for the prevention, treatment or reduction in severity of oral mucositis in a subject undergoing cancer therapy, comprising administering to the subject a therapeutically effective amount of a compound of Formula (I), a pharmaceutically acceptable salt, a solvate, a stereoisomer or a diastereoisomer thereof, wherein the cancer therapy is selected from radiation therapy, chemotherapy, hematopoietic stem cell transplantation, bone marrow transplantation or a combination thereof.

According to an embodiment of the present invention, the cancer therapy is radiation therapy, chemotherapy or a combination thereof.

Also disclosed is a method for the prevention, treatment or reduction in severity of oral mucositis in a subject undergoing cancer therapy, comprising administering to the subject a therapeutically effective amount of a compound of Formula (I), a pharmaceutically acceptable salt, a solvate, a stereoisomer or a diastereoisomer thereof, prior to cancer therapy, concurrently with the cancer therapy, after the cancer therapy or in between two cancer therapies.

Also disclosed is method for the prevention, treatment or reduction in severity of oral mucositis in a subject undergoing cancer therapy, comprising administering to the subject a therapeutically effective amount of a compound of Formula (I), a pharmaceutically acceptable salt, a solvate, a stereoisomer or a diastereoisomer thereof, prior to the cancer therapy.

Also disclosed is a method for the prevention, treatment or reduction in the severity of oral mucositis in a subject undergoing cancer therapy comprising administering to the subject a therapeutically effective amount of a compound of Formula (I), concurrently with the cancer therapy.

Also disclosed is a method for the prevention, treatment or reduction in the severity of oral mucositis in a subject undergoing cancer therapy comprising administering to the subject a therapeutically effective amount of a compound of Formula (I), after the cancer therapy.

Also disclosed is a method for the prevention, treatment or reduction in the severity of oral mucositis in a subject undergoing cancer therapy comprising administering to the subject a therapeutically effective amount of a compound of Formula (I), in between two cancer therapies.

According to an embodiment of the present invention, compound of formula (I) is to be administered to the subject after administration of chemotherapy and before administration of radiation therapy.

Dosage of the compound of Formula (I) depends on the mode of administration, body weight, age of the patient and other factors that are commonly considered by a skilled medical practitioner.

According to an embodiment of the present invention, the subject in need of the prevention, treatment or reduction in the severity of oral mucositis is a patient receiving therapy for the treatment of cancer, including both solid and haematological cancer.

According to an embodiment of the present invention, the subject in need of the prevention, treatment or reduction in the severity of oral mucositis is a patient receiving therapy for the treatment of cancer, wherein the cancer is selected from acute myeloid leukemia, acute lymphoid leukemia, chronic myeloid leukemia, Hodgkin's disease, multiple myeloma, non-Hodgkin's disease or head and neck cancer.

According to an embodiment of the present invention, the subject in need of the prevention, treatment or reduction in the severity of oral mucositis is a patient receiving therapy for the treatment of cancer, wherein the cancer is head and neck cancer.

Also disclosed is a method for the prevention, treatment or reduction in severity of oral mucositis in a subject undergoing cancer therapy for head and neck cancer, comprising administering to the subject a therapeutically effective amount of a compound of Formula (I), or a pharmaceutically acceptable salt, a solvate, a stereoisomer or a diastereoisomer thereof.

Also disclosed is a method for the prevention, treatment or reduction in severity of oral mucositis in a subject undergoing cancer therapy for head and neck cancer, comprising administering to the subject a therapeutically effective amount of a compound of Formula (I), or a pharmaceutically acceptable salt, a solvate, a stereoisomer or a diastereoisomer thereof, wherein the cancer therapy includes radiation therapy, chemotherapy or a combination thereof.

Also disclosed is a method for the prevention, treatment or reduction in the severity of oral mucositis in a subject undergoing radiation therapy for head and neck cancer, comprises administering a compound of Formula (I), wherein oral mucositis includes inflammation of the mucosal cells of the oral cavity. The other symptoms associated with oral mucositis include oral pain, mouth and throat sores, dysphagia (difficulty in swallowing) including feeding tube dependency, odynophagia (painful swallowing), lost or altered taste (dysgeusia), ulcers, nausea and vomiting, loss of appetite, fatigue, dehydration, weight loss, malnutrition and potential life threatening aspiration.

According to an embodiment of the present invention, the cumulative dose of the radiation administered to a subject in need thereof is from 30 Gy to 82 Gy.

According to an embodiment of the present invention, the cumulative dose of the radiation administered to a subject in need thereof is from about 50 Gy to about 82 Gy.

According to an embodiment of the present invention, the cumulative dose of the radiation administered to a subject in need thereof is from about 66 Gy to about 82 Gy.

According to an embodiment of the present invention, the cumulative dose of the radiation administered to a subject in need thereof is from about 66 Gy to about 75 Gy.

According to an embodiment of the present invention, the cumulative dose of the radiation administered to a subject in need thereof is from about 60 Gy to about 75 Gy.

According to an embodiment of the present invention, the cumulative dose of the radiation administered to a subject in need thereof is from about 60 Gy to about 70 Gy.

According to an embodiment of the present invention, the cumulative dose of the radiation administered to a subject in need thereof is 66 Gy.

According to an embodiment of the present invention, the cumulative dose of the radiation administered to a subject in need thereof is 60 Gy.

Also disclosed is a method for the prevention, treatment or reduction in the severity of oral mucositis in a subject undergoing cancer therapy for head and neck cancer, comprising administering compound of Formula (I) to a subject in need thereof, wherein the chemotherapeutic drug is a platinum-containing antineoplastic agent.

According to an embodiment of the present invention, the platinum-containing antineoplastic agent is cisplatin.

According to an embodiment of the present invention, the dose of cisplatin administered to a subject in need thereof, is from 30 mg/m² to 40 mg/m².

Further disclosed are the following methods:

A method for the prevention, treatment or reduction in the severity of oral mucositis in a subject undergoing cancer therapy for head and neck cancer comprising administering to the subject in need thereof 9 mg/m²/day to 259 mg/m²/day of the compound A.

A method for the prevention, treatment or reduction in the severity of oral mucositis in a subject undergoing cancer therapy for head and neck cancer comprising administering to the subject in need thereof 9 mg/m²/day to about 185 mg/m²/day of the compound A.

A method for the prevention, treatment or reduction in the severity of oral mucositis in a subject undergoing cancer therapy for head and neck cancer comprising administering to the subject in need thereof about 50 mg/m²/day to about 100 mg/m²/day of the compound A.

A method for the prevention, treatment or reduction in the severity of oral mucositis in a subject undergoing cancer therapy for head and neck cancer comprising administering to the subject in need thereof about 100 mg/m²/day of the compound A.

A method for the prevention, treatment or reduction in the severity of oral mucositis in a subject undergoing radiation therapy for head and neck cancer comprises the steps of: (a) administering compound A to the subject intravenously; and (b) administering radiation therapy within 1.5 to 2 hours after the infusion of compound A.

A method for the prevention, treatment or reduction in the severity of oral mucositis in a subject undergoing radiation therapy for head and neck cancer, comprises the steps of: (a) administering intravenously 9 mg/m²/day to 259 mg/m²/day of compound A to the subject; and (b) administering about 60 to about 70 Grays of cumulative radiation therapy within 1.5 to 2 hours after the infusion of compound A.

A method for the prevention, treatment or reduction in the severity of oral mucositis in a subject undergoing radiation therapy for head and neck cancer, comprises the steps of: (a) administering intravenously 9 mg/m²/day to about 185 mg/m²/day of compound A to the subject; and (b) administering about 60 to about 70 Grays of cumulative radiation therapy within 1.5 to 2 hours after the infusion of compound A.

A method for the prevention, treatment or reduction in the severity of oral mucositis, in a subject undergoing radiation therapy for head and neck cancer, comprises the steps of: (a) administering intravenously about 100 mg/m²/day of compound A to the subject; and (b) administering about 1.8 Grays to about 2 Grays/day of radiation therapy immediately after the infusion of compound A.

A method for the prevention, treatment or reduction in the severity of oral mucositis in a subject undergoing radiation therapy for head and neck cancer comprises the steps of: (a) administering cisplatin to the subject intravenously (b) administering compound A to the subject intravenously; and (c) administering radiation therapy within 2 hours after the end of the infusion of compound A.

A method for the prevention, treatment or reduction in the severity of oral mucositis in a subject undergoing radiation therapy for head and neck cancer comprises the steps of: (a) administering intravenously about 30 mg/m² to about 40 mg/m² of cisplatin (b) administering intravenously about 100 mg/m²/day of compound A to the subject; and (c) administering from about 66 Grays to about 82 Grays of cumulative radiation therapy within 2 hours after the end of the infusion of compound A.

A method for the prevention, treatment or reduction in the severity of oral mucositis, in a subject undergoing a combination of chemotherapy and radiation therapy for head and neck cancer, comprises the steps of: (a) administering intravenously about 30 mg/m² to about 40 mg/m² of cisplatin (b) administering intravenously about 100 mg/m²/day of compound A to the subject on days 1 to 5 of weeks 1, 4 and 7 and (c) administering about 1.8 Grays to about 2 Grays/day of radiation therapy within 2 hours after the infusion of compound A.

According to the present invention, the subject in need of treatment of head and neck cancer may be administered with radiation therapy of the type: Internal Radiation Therapy, External Beam Radiation Therapy (EBRT), Three-dimensional Conformal Radiation Therapy (3D-CRT) or Intensity Modulated Radiotherapy (IMRT). EBRT involves the administration of radiation via a machine capable of producing high-energy external beam radiation. The radiation can be either electromagnetic (X-ray or gamma radiation) or particulate (α or β particles). Internal radiation therapy (brachytherapy), involves implantation of a radioactive isotope as the source of the radiation. 3D-CRT is an advanced type of external beam radiation therapy technique that targets the prescribed radiation dose to the tumor, contouring the spatial distribution of the dose to the precise 3D configuration of the tumor. IMRT is an advanced type of high-precision external beam radiation therapy. It improves the ability to conform the treatment volume to concave tumor shapes by creating a shaped radiation beam and delivering high doses of radiation to the tumor and significantly smaller doses of radiation to the surrounding normal tissues.

According to an embodiment of the present invention, the subject in need of treatment for head and neck cancer may be administered with external beam radiation therapy.

There is provided a pharmaceutical composition which comprises a therapeutically effective amount of compound of Formula (I), a pharmaceutically acceptable salt, a solvate, a stereoisomer, or a diastereoisomer thereof; in association with a pharmaceutically acceptable carrier for use in the prevention, treatment or reduction in the severity of oral mucositis.

There is provided a pharmaceutical composition which comprises a therapeutically effective amount of compound of Formula (IA), a pharmaceutically acceptable salt, a solvate, a stereoisomer, or a diastereoisomer thereof in association with a pharmaceutically acceptable carrier for use in the prevention, treatment or reduction in the severity of oral mucositis.

The pharmaceutical preparations/compositions may contain about 1% to 99%, for example, about 5% to 70%, or from about 5% to about 30% by weight of the compound of the Formula (I) or pharmaceutically acceptable salt thereof as the active ingredient. The amount of the compound of the Formula (I) or pharmaceutically acceptable salt thereof in the pharmaceutical preparations normally is from about 1 mg to 1000 mg.

Administration of the pharmaceutical composition containing the compound of Formula (I) disclosed herein may be via any route known to be effective to a skilled medical practitioner. The compound of Formula (I) may be administered orally, topically or parenterally (including intravenous, subcutaneous, intramuscular, intravascular or infusion).

Compositions intended for pharmaceutical use may be prepared according to any method known in the art for the manufacture of pharmaceutical compositions, e.g. Remington - The Science and Practice of Pharmacy (21st Edition) (2005), Goodman & Gilman's The Pharmacological Basis of Therapeutics (11th Edition) (2006) and Ansel's Pharmaceutical Dosage Forms and Drug Delivery Systems (9th Edition), edited by Allen et al., Lippincott Williams & Wilkins, (2011), Solid-State Chemistry of Drugs (2nd Edition)(1999), each of which is hereby incorporated by reference."

The compositions described herein may be in a form suitable for oral administration, for example, solid dosage forms such as tablets, capsules, lozenges, or granules; liquid dosage forms such as, emulsions, solutions, suspensions; for parenteral injection (including intravenous, subcutaneous, intramuscular, intravascular or infusion) for example as a sterile solution, suspension or emulsion; for topical administration for example as an ointment, cream, gel, lotions or collodion.

Compositions for oral delivery may be in the form of tablets, lozenges, aqueous or oily suspensions, granules, powders, cachets, emulsions, capsules, syrups, or elixirs. Orally administered compositions may contain one or more optional agents, for example, sweetening agents such as fructose, aspartame or saccharin; flavoring agents such as peppermint, oil of wintergreen, or cherry; coloring agents; and preserving agents, to provide a pharmaceutically palatable preparation. Selectively permeable membranes surrounding an osmotically active driving compound are also suitable for oral administration of compounds of present invention. Oral compositions can include standard vehicles such as mannitol, lactose, starch, corn starch, magnesium stearate, talc, sodium saccharine, cellulose, magnesium carbonate, etc. Such vehicles are preferably of pharmaceutical grade.

For ointments, creams, the compound of Formula (I) is formulated in oil-in-water or water-in-oil base.

For intramuscular, intraperitoneal, subcutaneous and intravenous use, sterile solutions of compound of Formula (I) are usually employed, and the pH of the solutions should be suitably adjusted and buffered.

Further, the effect of the compounds of Formula (I) contained in the pharmaceutical composition may be delayed or prolonged by proper formulation. For example, a slowly soluble pellet of the compound may be prepared and incorporated in a tablet or capsule. The technique may be improved by making pellets of several different dissolution rates and filling capsules with a mixture of the pellets. Tablets or capsules may be coated with a film which resists dissolution for a predictable period of time.

Even the parenteral preparations may be made long-acting, by dissolving or suspending the compound in oily or emulsified vehicles which allow it to disperse only slowly in the serum.

Effective dose of the compound of Formula (I) depends at least on the nature of the condition being treated, the mode of delivery, and the pharmaceutical formulation, and will be determined by the skilled medical practitioner or clinician using conventional dose escalation studies. It can be from 9 mg/m² to 259 mg/m² per day; particularly, from about 9 mg/m² to about 185 mg/m² per day; more particularly, from about 50 mg/m² to about 100 mg/m² per day.

Compounds of Formula (I) may be prepared according to the methods disclosed in PCT Patent Publication No. WO2004004632 and PCT Patent Publication No. WO2007148158.

The general process for the preparation of compounds of Formula (I), or a pharmaceutically acceptable salt thereof, comprises the following steps:
(a) treating the resolved enantiomerically pure (-)-trans enantiomer of the intermediate compound of Formula VIA, 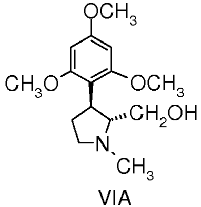 with acetic anhydride in the presence of a Lewis acid catalyst to obtain a resolved acetylated compound of Formula VIIA, 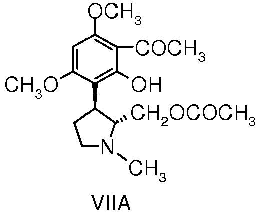
(b) reacting the resolved acetylated compound of Formula VIIA with an acid of Formula ArCOOH or an acid chloride of Formula ArCOCl or an acid anhydride of Formula (ArCO)₂O or an ester of Formula ArCOOCH₃, wherein Ar is as defined hereinabove in reference to the compound of Formula (I), in the presence of a base and a solvent to obtain a resolved compound of Formula VIIIA; 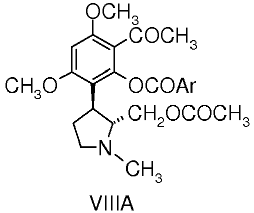
(c) treating the resolved compound of Formula VIIIA with a base in a suitable solvent to obtain the corresponding resolved β-diketone compound of Formula IXA; 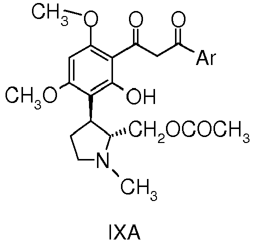 wherein Ar is as defined above;
(d) treating the resolved β-diketone compound of Formula IXA with an acid such as hydrochloric acid to obtain the corresponding cyclized compound of Formula XA, 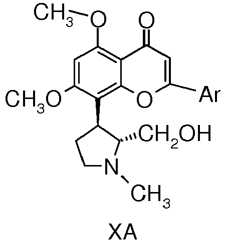
(e) subjecting the compound of Formula XA to dealkylation by heating it with a dealkylating agent at a temperature ranging from 120-180 °C to obtain the (+)-*trans* enantiomer of the compound of Formula (I) and, optionally, converting the subject compound into its pharmaceutically acceptable salt.

The Lewis acid catalyst utilized in the step (a) above may be selected from: BF₃, Et₂O, zinc chloride, aluminium chloride and titanium chloride.

The base utilized in the process step (b) may be selected from triethylamine, pyridine and a DCC-DMAP combination (combination of N, N'-dicyclohexyl carbodiimide and 4-dimethylaminopyridine).

It will be apparent to those skilled in the art that the rearrangement of the compound of Formula VIIIA to the corresponding β-diketone compound of Formula IXA is known as a *Baker-Venkataraman* rearrangement (J. Chem. Soc., 1933, 1381 and Curr. Sci., 1933, 4, 214).

The base used in the process step (c) may be selected from: lithium hexamethyl disilazide, sodium hexamethyldisilazide, potassium hexamethyldisilazide, sodium hydride and potassium hydride. A preferred base is lithium hexamethyl disilazide.

The dealkylating agent used in process step (e) for the dealkylation of the compound of Formula IXA may be selected from: pyridine hydrochloride, boron tribromide, boron trifluoride etherate and aluminium trichloride. A preferred dealkylating agent is pyridine hydrochloride.

Preparation of the starting compound of Formula VIA involves reacting 1-methyl-4-piperidone with a solution of 1,3,5-trimethoxybenzene in glacial acetic acid, to yield 1-methyl-4-(2,4,6-trimethoxyphenyl)-1,2,3,6-tetrahydropyridine, which is reacted with boron trifluoride diethyl etherate, sodium borohydride and tetrahydrofuran to yield 1-methyl-4-(2,4,6-trimethoxyphenyl)piperidin-3-ol. Conversion of 1-methyl-4-(2,4,6-trimethoxyphenyl)piperidin-3-ol to the compound of Formula VIA involves converting the hydroxyl group present on the piperidine ring of the compound, 1-methyl-4-(2,4,6-trimethoxyphenyl) piperidin-3-ol to a leaving group such as tosyl, mesyl, triflate or halide by treatment with an appropriate reagent such as p-toluenesulfonylchloride, methanesulfonylchloride, triflic anhydride or phosphorous pentachloride in the presence of oxygen nucleophiles such as triethylamine, pyridine, potassium carbonate or sodium carbonate, followed by ring contraction in the presence of oxygen nucleophiles such as sodium acetate or potassium acetate in an alcoholic solvent such as isopropanol, ethanol or propanol.

Each numerical parameter should at least be construed in light of the number of reported significant digits and by applying ordinary rounding techniques.

The invention will now be described in greater detail by reference to the following non-limiting examples. The following examples further illustrate the invention.

### EXEMPLIFICATION

In the following examples and elsewhere, abbreviations have the following meanings:

| List of abbreviations | | | |
|---|---|---|---|
| BF₃ | boron trifluoride | Mm | millimetres |
| cm | centimetres | MeOH | methanol |
| e.e | enantiomeric excess | Mg | milligram |
| Et₂O | diethyl ether | mL | milliliter |
| °F | Degree Fahrenheit | Mm | millimetre |
| g | gram | mmol or mM | millimolar |
| h | hour(s) | m² | square meter |
| HCl | hydrochloric acid | nm | nanometers |
| HPLC | high performance liquid chromatography | Na₂CO₃ | sodium carbonate |
| IPA | isopropyl alcohol | TFA | trifluoroacetic acid |

### Reference example 1

### A) Preparation of (+)-trans-2-(2-Chlorophenyl)-5,7-dihydroxy-8-(2-hydroxymethyl-1-methyl-pyrrolidin-3-yl)-chromen-4-one hydrochloride (Compound A)

Molten pyridine hydrochloride (4.1 g, 35.6 mmol) was added to (+)-trans-2-(2-chloro-phenyl)-8-(2-hydroxymethyl-1-methyl-pyrrolidin-3-yl)-5,7-dimethoxy-chromen-4-one (0.4 g, 0.9 mmol) and heated at 180 °C for 1.5 h. The reaction mixture was cooled to 25 °C, diluted with MeOH (10 mL) and basified using Na₂CO₃ to pH 10. The mixture was filtered and the organic layer was concentrated. The residue was suspended in water (5 mL), stirred for 30 minutes filtered and dried to obtain the compound, (+)-trans-2-(2-chloro-phenyl)-5,7-dihydroxy-8-(2-hydroxymethyl-1-methyl-pyrrolidin-3-yl)-chromen-4-one.
Yield: 0.25 g (70 %); IR (KBr): 3422, 3135, 1664, 1623, 1559 cm⁻¹; 1 H NMR (CDCl₃, 300MHz): δ 7.56 (d, 1 H), 7.36 (m, 3H), 6.36 (s, 1 H), 6.20 (s, 1 H), 4.02 (m, 1 H), 3.70 (m, 2H), 3.15 (m, 2H), 2.88 (m, 1 H), 2.58 (s, 3H), 2.35 (m, 1 H), 1.88 (m, 1H); MS (ES+): m/z 402 (M+1);
Analysis: C₂₁H₂₀ClNO₅; C, 62.24 (62.71); H, 5.07 (4.97); N, 3.60 (3.48); Cl, 9.01 (8.83).

The compound (0.2 g, 0.48 mmol) as obtained above was suspended in IPA (5 mL) and 3.5 % HCl (25 mL) was added. The suspension was heated to get a clear solution. The solution was cooled and solid filtered to obtain the compound, (+)-trans-2-(2-Chlorophenyl)-5,7-dihydroxy-8-(2-hydroxymethyl-1-methyl-pyrrolidin-3-yl)-chromen-4-one hydrochloride.

Yield: 0.21 g (97 %); mp: 188 - 192 °C; [α]D25 = +21.3°(c = 0.2, methanol); 1 H NMR (CD₃OD, 300MHz): δ 7.80 (d, 1 H), 7.60 (m, 3H), 6.53 (s, 1 H), 6.37 (s, 1 H), 4.23 (m, 1 H), 3.89 (m, 2H), 3.63 (m, 1 H), 3.59 (dd, 1 H), 3.38 (m, 1 H), 2.90 (s, 3H), 2.45 (m, 1 H), 2.35 (m, 1 H); MS (ES+): m/z 402 (M +1)(free base).

This compound was subjected to chiral HPLC. Chiral HPLC was done using column Chiralcel OD-H (250 x 4.6 mm) and solvent system haxane:ethanol (92:08) with TFA (0.4%). The results are recorded at 264nm with solvent flow rate of 1 mL/minute The chiral HPLC showed 100% e.e of the compound, (+)-trans-2-(2-chloro-phenyl)-5,7-dihydroxy-8-(2-hydroxy-methyl-1-methyl-pyrrolidin-3-yl)-chromen-4-one hydrochloride.

### B) Preparation of (+)-trans-2-(2-chloro-4-trifluoromethyl-phenyl)-5,7-dihydroxy-8-(2-hydroxymethyl-1-methyl-pyrrolidin-3-yl)-chromen-4-one hydrochloride (Compound B)

A mixture of the compound, (+)-trans-2-(2-Chloro-4-trifluoromethylphenyl)-8-(2-hydroxymethyl-1-methyl pyrrolidin-3-yl)-5,7-dimethoxy-chromen-4-one (0.25 g, 0.5 mmol), pyridine hydrochloride (0.25 g, 2.16 mmol) and a catalytic amount of quinoline was heated at 180 °C for a period of 2.5 h. The reaction mixture was diluted with methanol (25 mL) and basified with solid Na₂CO₃ to pH 10. The reaction mixture was filtered, and washed with methanol. The organic layer was concentrated and the residue purified by column chromatography using 0.1 % ammonia and 4.5 % MeOH in chloroform as eluent to yield the compound, (+)-trans-2-(2-chloro-4-trifluoromethylphenyl)-5,7-dihydroxy-8-(2-hydroxy-methyl-1-methylpyrrolidin-3-yl)-chromen-4-one, as a yellow solid.
Yield: 0.15 g (63.7 %); 1 H NMR (CDCl₃, 300MHz): δ 7.99 (m, 2H), 7.83 (d, 1 H), 6.65 (s, 1 H), 6.41 (s, 1 H), 4.24 (m, 1 H), 3.90 (m, 2H), 3.70 (m, 1 H), 3.60 (m, 1 H), 3.41 (m, 1 H), 2.99 (s, 3H), 2.54 (m, 1 H), 2.28 (m, 1 H); MS (ES+): m/z 470 (M+1).

The compound (0.1 g, 0.2 mmol) as obtained above was suspended in methanol (2 mL) and treated with ethereal HCl and the organic solvent evaporated to yield the compound, (+)-trans-2-(2-chloro-4-trifluoromethyl-phenyl)-5,7-dihydroxy-8-(2-hydroxymethyl-1-methyl-pyrrolidin-3-yl)-chromen-4-one hydrochloride.
Yield: 0.1 g (92.8 %); 1 H NMR (CDCl₃, 300MHz): δ 8.02 (d, 2H), 7.83 (d, 1 H), 6.64 (s, 1 H), 6.41 (s, 1 H), 4.23 (m, 1 H), 3.73 (m, 2H), 3.68 (m, 1 H), 3.51 (m, 1 H), 3.39 (m, 1 H), 2.99 (s, 3H), 2.54 (m, 1 H), 2.31 (m, 1 H).

### PHARMACOLOGY

### Example 1: To assess the effect of compound A in the prevention and/or treatment of radiation induced oral mucositis in Hamsters

The experiment was carried out at Biomodel's facility in Watertown, Massachusetts, USA. IACUC (Institutional Animal Care and Use Committee) approval for this study (10-0614-06) was obtained from Biomodel's IACUC.

### Materials and Methods:

### Animals:

Male LVG Syrian Golden Hamsters, 5 to 6 weeks old, weighing 87.7 to 97.1 g (Charles River Laboratories, US) at the commencement of the study, were used. An ear punch was used to number the animals individually. Approximately 8-10 animals were housed per cage. The animals were acclimatized for at least 3 days before experimentation. During this period, they were observed daily to ensure that the animals are in good condition. The animals that presented poor condition were rejected.

Animal room housing the aforesaid animals was provided with filtered air at a temperature of 65 to 75 °F and 30 to 70% humidity. Animal room received a minimum of 12 to 15 air changes per hour and was maintained on an automatic timer for a light/dark cycle of 12 hours each with no twilight. Animals were fed with Purina Labdiet® 5053 sterile rodent chow and soft food. Sterile water was provided ad *libitum.*

### Conditions for storage of the compounds and Dose preparation

Compound A: 5 mg/mL and 10 mg/mL; vehicle: dextrose (5%) prepared in water. All the compounds including the standard were stored at 2 °C to 8 °C.

### Animal Randomization and Allocation:

Animals were randomly and prospectively divided into 5 groups:
i) Group 1: Animals (n=8) were administered with 5% w/v dextrose monohydrate intraperitoneally from Day 0 to Day 4 or from Day 0 to Day 9.
ii) Group 2: Animals (n=10) were administered with 10 mg/kg of compound A intraperitoneally from Day 0 to Day 4.
iii) Group 3: Animals (n=10) were administered with 20 mg/kg of compound A intraperitoneally from Day 0 to Day 4.
iv) Group 4: Animals (n=10) administered with 10 mg/kg of compound A intraperitoneally from Day 0 to Day 9.
v) Group 5: Animals (n=10) administered with 20 mg/kg of compound A intraperitoneally from day Day 0 to Day 9.

### Treatment:

Mucositis induction: The animals were anesthetized and the left buccal pouch was everted, fixed and isolated using a lead shield. The left buccal pouch mucosa of the animal was exposed to radiation at a rate of 2.0 Gy/minute. A single dose of radiation (40 Gy/dose) was administered to all animals on Day 0. Radiation was generated with 160 kilovolt potential source at a focal distance of 21 cm, hardened with a 0.35 mm Al filtration system. Animals of Group 2 to 5 were treated with compound A as per the schedule detailed above one hour prior to the radiation. The volume administered to all the above Groups was 0.2 mL/100g.

### Mucositis:

For evaluation of mucositis, the animals were anesthetized with an inhalation anesthetic and the left buccal pouch everted. Mucositis was scored visually using the following criteria for evaluation:

| Score | Description |
|---|---|
| 0 | Pouch completely healthy. No erythema or vasodilation. |
| 1 | Light to severe erythema and vasodilation. No erosion of mucosa. |
| 2 | Severe erythema and vasodilation. Erosion of superficial aspects of mucosa leaving denuded areas. Decreased stippling of mucosa. |
| 3 | Formation of off-white ulcers in one or more places. Ulcers may have a yellow/ gray color due to pseudomembrane. Cumulative size of ulcers should equal less than or equal to 1/4^{th} of the pouch. Severe erythema and vasodilation. |
| 4 | Cumulative size of ulcers should equal about 1/2 of the pouch. Loss of pliability. Severe erythema and vasodilation. |
| 5 | Virtually all of pouch is ulcerated. Loss of pliability (pouch can only partially be extracted from mouth). |

Weight change and survival were also measured throughout the study.

### Observations and Results:

### Survival:

Three deaths occurred during the study. Two deaths were attributed to the use of anesthesia during radiation. One animal was sacrificed on Day 19, upon development of fistula that formed on the abdomen following ulceration of the injection site.

### Weight Change:

The one-way ANOVA test revealed that there was no significant difference in percent body weight change among groups.

### Mucositis:

Mean daily mucositis scores for each group were evaluated. The maximum mean mucositis score observed in the vehicle control group was 3.0, which occurred on Day 16. The treatment Groups 2 and 3 had maximum mean mucositis scores of 3.0 and 3.0 on Day 18 and Day 16, respectively. The treatment Groups 4 and 5 had maximum mean mucositis scores on Day 16 with average scores of 3.0 and 3.1 respectively.

### Duration of Ulcerative Mucositis:

The significance of differences observed between the different treatment groups was evaluated by comparing the days with mucositis scores ≥ 3 and ≤ 3 between groups using a chi-squared analysis.

Result: For the entire duration of the study, the percentage of animal days with a score of ≥ 3 in the vehicle control group was 63.0%. The percentage of days with a score of ≥ 3 was significantly reduced to 43.5% in Group 4. The percentage of animal days with a score of ≥ 3 for the other 3 treatment groups was not significantly different compared to the vehicle control group.

### Mucositis Severity:

An analysis of the severity of mucositis was performed using Mann-Whitney rank sum analysis to compare the visual mucositis scores for each treatment group to the vehicle control on each day of the analysis.

Result: The treatment Group 4 showed significant reductions in mucositis on Day 24 (p=0.002) and Day 26 (p=0.024) and also it showed strong trend of lower scores on Day 18 (p=0.073) and Day 28 (p=0.064) compared to the vehicle control i.e Group 1.

### Percentage of Animals with Ulcerative Mucositis by Day

The percentage of animals in each group with ulcerative mucositis at each day of the study was evaluated.

Result: The treatment Group 4 showed notably low percentage of ulceration by Day with Mucositis Score ≥ 3. On Days 24 to 28 of the study the percentage of animals with ulcerative mucositis was reduced significantly by 72.7%, 83.8 and 77.8% respectively, compared to the Group 1.

### Conclusions:

1. Treatment with Compound A at 10 mg/kg from Days 0 to 9 significantly reduced the percent of days and the number of days with ulcerative mucositis (score ≥3) compared to the vehicle control Group.
2. Treatment with Compound A at 10 mg/kg from Days 0 to 9 resulted in a significant improvement in mucositis scores on Day 24 and 26 of the study compared to the vehicle control Group.

### Example 2: To assess the effect of compound A in the prevention and/or treatment of radiation induced oral mucositis

### Clinical Study

The clinical study was carried out by the method described below:

### Patient selection:

A total of 23 patients with squamous cell carcinoma of oral cavity, oropharynx and hypopharynx were enrolled in the clinical study of compound A. Of the 23 patients, 19 patients underwent a complete treatment schedule and hence 19 patients were evaluated for efficacy.

### Treatment Schedule:

The compound A and external beam radiotherapy (EBRT) were administered for six weeks i.e., 2 cycles of compound A and 60 fractions of radiation. One, three weeks (21 days) cycle of combination regimen comprised of dosing compound A for days 1 to 5 and EBRT on days 1 to 5, 8 to 12 and 15 to 19. The combination treatment schedule is depicted in the following table:

| Cycle 1 | | | | | | |
|---|---|---|---|---|---|---|
| | Day 1-5 | Day 6-7 | Day 8-12 | Day 13-14 | Day 15-19 | Day 20-21 |
| Compound A | ✔ | × | × | × | × | × |
| Radiation | ✔ | × | ✔ | × | ✔ | × |

| Cycle 2 | | | | | | |
|---|---|---|---|---|---|---|
| | Day 1-5 | Day 6-7 | Day 8-12 | Day 13-14 | Day 15-19 | Day 20-21 |
| Compound A | ✔ | × | × | × | × | × |
| Radiation | ✔ | × | ✔ | × | ✔ | × |

The tick mark symbol (✔) indicates that compound A or radiation, as applicable is administered to the subject.

The cross symbol (×) indicates that compound A or radiation, as applicable, is not administered to the subject.

### Treatment Procedure:

A] Dose of compound A: In the Phase I part of the clinical trial, 11 patients were enrolled and 100 mg/m²/day of compound A was determined to be the maximum tolerated dose (MTD) as two dose limiting toxicities occurred at the higher dose of 140 mg/m²/day. Additional 12 subjects were enrolled at the MTD dose level to confirm the safety and tolerability of that dose.
B] Procedure: Compound A (100 mg/m²/day) in 5% dextrose (200 mL) was administered to 19 patients as intravenous infusion over 30 minutes on days 1 to 5 of a 21 day cycle. After about 1.5 hours of infusion of compound A, the patients received EBRT (2 Grays per day for 5 days) to the affected body parts (primary tumor site and involved cervical lymph nodes) through linear accelerator. Patients continued to receive EBRT (2 Grays per day) on days 8-12 and 15-19. On days 6, 7, 13 and 14 patients were not infused with compound A, neither were exposed to radiotherapy.

The procedure was repeated for cycle 2.

At investigator's discretion, some patients were given additional radiation of up to 10 additional Grays (2 Grays per day for 5 days) commencing immediately after the completion of the prescribed radiation dose of 60 Grays. Total radiation dose for spinal cord was less than 48 Grays.

The radiation breaks were compensated by delivering the missed doses over weekends or an additional dose on the next day with a minimum gap of 6 hours between two radiation doses or immediately after the end of cycle 2.

### Evaluation of toxicity criteria for oral mucositis in radiation therapy:

The Common Terminology Criteria for Adverse Events (version 3) (version 3, Publish Date: August 09, 2006) laid down by the National Cancer Institute for oral mucositis in radiation therapy was followed. The criteria for evaluation were as follows:

| Grade | | Adverse Event |
|---|---|---|
| 1 | (mild) | Erythema of the mucosa |
| 2 | (moderate) | Patchy ulcerations or pseudomembranes |
| 3 | (severe) | Confluent ulcerations or pseudomembranes; bleeding with minor trauma |
| 4 | (severe) | Tissue necrosis; significant spontaneous bleeding; life threatening consequences |
| 5 | | Death |

Observations and Results: After completion of the trial, it was found that only five events of mucositis were reported in the 19 evaluable patients:
a) one severe mucositis (Grade 3),
b) two moderate stomatitis (Grade 2),
c) one moderate mucositis (Grade 2),
d) one mild mucositis (Grade 1).

The results show that the compound A has radioprotective effects and compares favourably with historical rates of serious RIM (Grade 3 and above) as reported by Trotti et al., in Radiotherapy and Oncology, 2003, 66, 253-262.

### Example 3: To assess the effect of compound A in the prevention and/or treatment of chemo-radiotherapy induced oral mucositis

### Protocol 1

### Patient selection:

A total of 60 patients with locally advanced squamous cell carcinoma of head and neck (SCCHN), will be enrolled in the clinical study of compound A.

### Treatment Schedule:

Compound A and external beam radiotherapy (EBRT) will be administered to the patients for seven weeks. Cisplatin will be administered intravenously on Day 1 or Day 2 of every week. Compound A will be administered over 30 minutes on Days 1 to 5 of weeks 1, 4 and 7. The patients will be irradiated within 2 hours after the end of the infusion of Compound A.

The patients treated with cisplatin and Compound A are scheduled to receive at least 66 Gy of cumulative radiation.

A typical combination treatment schedule for seven weeks comprising of dosing of cisplatin as chemotherapeutic agent, compound A and EBRT is depicted in the following table:

**Table 1:**

| Week → Treatment↓ | Week 1 | Week 2 | Week 3 | Week 4 | Week 5 | Week 6 | Week 7 |
|---|---|---|---|---|---|---|---|
| Cisplatin | Day 1 or Day 2 | Day 1 or Day 2 | Day 1 or Day 2 | Day 1 or Day 2 | Day 1 or Day 2 | Day 1 or Day 2 | Day 1 or Day 2 |
| Compound A | Day 1-5 | × | × | Day 1-5 | × | × | Day 1-5 |
| Radiation | Day 1-5 | Day 1-5 | Day 1-5 | Day 1-5 | Day 1-5 | Day 1-5 | Day 1-5 |

The cross symbol (×) indicates that compound A will not be administered to the subject.

### Treatment Procedure:

A] Dose of compound A: 100 mg/m²/day of compound A is determined to be the maximum tolerated dose (MTD).
B] Procedure: Cisplatin will be administered at a dose of 30 to 40 mg/m² (the selection of the dose is left open to investigator and institutional standards) on the first or second day of week 1 of the treatment and then will be administered on the weekly basis the same day of the week in the subsequent weeks (week 2 to 7). Compound A (100 mg/m²/day) in 5% dextrose will be administered as intravenous infusion over 30 minutes on days 1 to 5 of weeks 1, 4 and 7. Within 2 hours of infusion of compound A, the patients will be exposed to EBRT to the involved body parts (primary tumor site and grossly involved cervical lymph nodes and sub-clinical lymph nodes) by using standard conventional fractionation of 2 Grays per day for 5 days per week for a total radiation dose of at least 66 Gy over 7 weeks.

The radiation breaks will be compensated by delivering the missed doses over weekends or an additional dose on the next day with a minimum gap of 6 hours between two radiation doses or immediately after the end of the week 7 treatment.

### Evaluation of Toxicity criteria for oral mucositis in radiation therapy:

The WHO Toxicity Criteria was followed (WHO Handbook for Reporting Results of Cancer Treatment).

The criteria for evaluation were as follows:

| Grade | Scale |
|---|---|
| 0 | None |
| 1 | Soreness and Erythema; no ulcers |
| 2 | Ulcers; able to eat a solid diet |
| 3 | Ulcers; requires a liquid diet |
| 4 | Ulcers; not able to tolerate a solid or liquid diet; requires IV or tube feeding. |

The following parameters will be evaluated:
1. The incidence of severe radiation induced mucositis (WHO Grade ≥ 3) occurring upto a cumulative radiation dose of 66 Gy.
2. The time for onset of severe radiation induced mucositis (WHO Grade ≥ 3) from the start of study treatment i.e. the number of days between start of study treatment and the first time that WHO Grade 3 or 4 mucositis is observed.
3. The duration of severe radiation induced mucositis (WHO Grade ≥ 3) i.e. the number of days from the onset of severe radiation induced mucositis to the day when severe radiation induced mucositis is resolved (WHO Grade ≤ 3).
4. Locoregional control.
5. Progression-free survival.
6. Overall survival.
7. Safety and tolerability of the combination regimen of compound A with radiation and cisplatin.

### Protocol 2:

The protocol for the clinical study is described below:

### Patient selection:

An appropriate number of patients with locally advanced squamous cell carcinoma of head and neck (SCCHN), will be enrolled in the clinical study of compound A.

### Treatment Schedule:

The study will be carried out in two different treatment arms, wherein the patients will be scheduled to receive:
Treatment arm 1: Cisplatin, compound A and radiation therapy or
Treatment arm 2: Cisplatin and radiation therapy.

Compound A (for treatment arm 1) and external beam radiotherapy (EBRT) will be administered to the patients for seven weeks. Cisplatin will be administered intravenously on Day 1 or Day 2 of every week. Compound A (for treatment arm 1) will be administered over 30 minutes on Days 1 to 5 of weeks 1, 4 and 7. The patients will be irradiated within 2 hours after the end of the infusion of Compound A.

A typical combination treatment schedule for seven weeks comprising of dosing of cisplatin as chemotherapeutic agent, compound A and EBRT is depicted in the following tables:

**Table 2: Treatment Arm 1**

| Week → Treatment↓ | Week 1 | Week 2 | Week 3 | Week 4 | Week 5 | Week 6 | Week 7 |
|---|---|---|---|---|---|---|---|
| Cisplatin | Day 1 or Day 2 | Day 1 or Day 2 | Day 1 or Day 2 | Day 1 or Day 2 | Day 1 or Day 2 | Day 1 or Day 2 | Day 1 or Day 2 |
| Compound A | Day 1-5 | × | × | Day 1-5 | × | × | Day 1-5 |
| Radiation | Day 1-5 | Day 1-5 | Day 1-5 | Day 1-5 | Day 1-5 | Day 1-5 | Day 1-5 |

The cross symbol (×) indicates that compound A is not administered to the patient.

**Table 3: Treatment Arm 2**

| Week → Treatment↓ | Week 1 | Week 2 | Week 3 | Week 4 | Week 5 | Week 6 | Week 7 |
|---|---|---|---|---|---|---|---|
| Cisplatin | Day 1 or Day 2 | Day 1 or Day 2 | Day 1 or Day 2 | Day 1 or Day 2 | Day 1 or Day 2 | Day 1 or Day 2 | Day 1 or Day 2 |
| Radiation | Day 1-5 | Day 1-5 | Day 1-5 | Day 1-5 | Day 1-5 | Day 1-5 | Day 1-5 |

### Treatment Procedure:

A] Dose of compound A: 100 mg/m²/day of compound A is determined to be the maximum tolerated dose (MTD).
B] Procedure: Cisplatin will be administered at a dose of 30 to 40 mg/m² (the selection of the dose is left open to investigator and institutional standards) on the first or second day of week 1 of the treatment and then given on the weekly basis the same day of the week in subsequent weeks (week 2 to 7). Compound A (100 mg/m²/day), in 5% dextrose will be administered as intravenous infusion over 30 minutes on days 1 to 5 of weeks 1, 4 and 7, to the patients of treatment arm 1. Patients enrolled in treatment arm 2 will not receive compound A. Within 2 hours of infusion of compound A (treatment arm 1) or within 2 hours of administration of cisplatin (treatment arm 2), the patients will be exposed to EBRT to the involved body parts (primary tumor site and grossly involved cervical lymph nodes and sub-clinical lymph nodes) by using standard conventional fractionation of 2 Grays per day for 5 days per week for a total radiation dose of at least 66 Gy over 7 weeks.

The radiation breaks will be compensated by delivering the missed doses over weekends or an additional dose on the next day with a minimum gap of 6 hours between two radiation doses or immediately after the end of the week 7 treatment.

### Evaluation of Toxicity criteria for oral mucositis in radiation therapy:

The WHO Toxicity Criteria was followed (WHO Handbook for Reporting Results of Cancer Treatment).

The criteria for evaluation were as follows:

| Grade | Scale |
|---|---|
| 0 | None |
| 1 | Soreness and Erythema; no ulcers |
| 2 | Ulcers; able to eat a solid diet |
| 3 | Ulcers; requires a liquid diet |
| 4 | Ulcers; not able to tolerate a solid or liquid diet; requires IV or tube feeding. |

The following parameters will be evaluated:
1. The incidence of severe radiation induced mucositis (WHO Grade ≥ 3) occurring upto a cumulative radiation dose of 54 Gy.
2. The incidence of severe radiation induced mucositis (WHO Grade ≥ 3) occurring upto a cumulative radiation dose of 66 Gy.
3. The time for onset of severe radiation induced mucositis (WHO Grade ≥ 3) from the start of study treatment i.e. the number of days between start of study treatment and the first time the WHO Grade 3 or 4 mucositis was observed.
4. The duration of severe radiation induced mucositis (WHO Grade ≥ 3) i.e. the number of days from the onset of severe radiation induced mucositis to the day when severe radiation induced mucositis is resolved (WHO Grade ≤ 3).
5. Locoregional control
6. Progression-free survival
7. Overall survival
8. Safety and tolerability of the two treatment arms.

All publications and patent applications in this specification are indicative of the level of ordinary skill in the art to which this invention pertains.

## Claims

1. A compound of Formula (I), 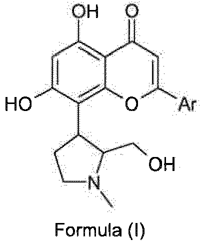 wherein Ar is a phenyl group substituted by 1, 2, or 3 identical or different substituents selected from chlorine, bromine, fluorine, iodine, C₁-C₄-alkyl and trifluoromethyl; or a pharmaceutically acceptable salt, a solvate, a stereoisomer or a diastereoisomer thereof, for use in the prevention, treatment or reduction in severity of oral mucositis in a subject undergoing cancer therapy.

2. The compound for the use according to claim 1, wherein the compound of Formula (I) is a (+)-trans isomer represented by Formula (IA), 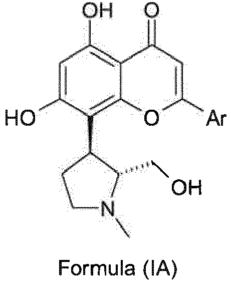 wherein Ar is a phenyl group, which is substituted by 1, 2, or 3 identical or different substituents selected from chlorine, bromine, fluorine, iodine, C₁-C₄-alkyl and trifluoromethyl; or a pharmaceutically acceptable salt or a solvate thereof.

3. The compound for the use according to claim 1 or claim 2, wherein the compound is administered to the subject, prior to cancer therapy, concurrently with the cancer therapy, after the cancer therapy or in between two cancer therapies.

4. The compound for the use according to claim 3, wherein the compound is to be administered to the subject prior to the cancer therapy.

5. The compound for the use according to claim 3, wherein the compound is to be administered to the subject concurrently with the cancer therapy.

6. The compound for the use according to claim 3, wherein the compound is to be administered to the subject after the cancer therapy.

7. The compound for the use according to claim 3, wherein the compound is to be administered to the subject in between two cancer therapies.

8. The compound for the use according to any one of the preceding claims 1 to 7, wherein the subject undergoing cancer therapy is a patient suffering from acute myeloid leukemia, acute lymphoid leukemia, chronic myeloid leukemia, Hodgkin's disease, multiple myeloma, non-Hodgkin's disease or head and neck cancer.

9. The compound of Formula (I) for the use according to claim 8, wherein the subject undergoing cancer therapy is a patient suffering from head and neck cancer.

10. The compound for the use according to any one of the preceding claims 1 to 9, wherein the compound is (+)-*trans-*2-(2-Chlorophenyl)-5,7-dihydroxy-8-(2-hydroxymethyl-1-methylpyrrolidin-3-yl)-chromen-4-one hydrochloride (compound A) or (+)-trans-2-(2-Chloro-4-trifluoromethyl-phenyl)-5,7-dihydroxy-8-(2-hydroxymethyl-1-methylpyrrolidin-3-yl)-chromen-4-one hydrochloride (compound B).

11. The compound for the use according to claim 10, wherein the compound A is to be administered in a dose from 9 mg/m²/day to 259 mg/m²/day.

12. The compound for the use according to any one of the preceding claims 1 to 11, wherein the cancer therapy is radiation therapy, chemotherapy or a combination thereof.

13. The compound for the use according to claim 12, wherein the cumulative dose of the radiation to be administered to a subject in need thereof is selected from 30 Grays to 82 Grays.

14. The compound for the use according to claim 13, wherein chemotherapy involves use of cisplatin, optionally wherein the dose of cisplatin to be administered to a subject in need thereof is from 30 mg/m² to 40 mg/m².

15. A pharmaceutical composition for use in the prevention, treatment or reduction in severity of oral mucositis in a subject undergoing cancer therapy, comprising a therapeutically effective amount of compound of Formula (I), 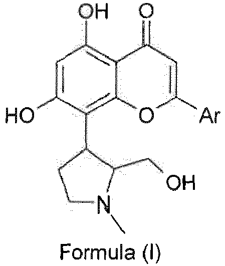 wherein Ar is a phenyl group substituted by 1 or 2 identical or different substituents selected from chlorine, bromine, fluorine, iodine, C₁-C₄-alkyl, and trifluoromethyl; or a pharmaceutically acceptable salt, a solvate, a stereoisomer or a diastereoisomer thereof and a pharmaceutically acceptable carrier.

## Patentansprüche

1. Verbindung der Formel (I), 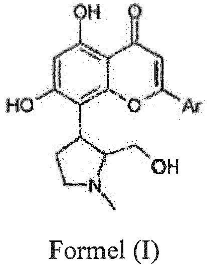 worin Ar Phenyl ist, das substituiert ist mit 1, 2 oder 3 identischen oder verschiedenen Substituenten ausgewählt unter Chlor, Brom, Fluor und Iod, C₁-C₄-Alkyl und Trifluormethyl, oder ein pharmazeutisch annehmbares Salz, ein Solvat, ein Stereoisomer oder ein Diastereoisomer davon, zur Verwendung bei der Verhinderung, Behandlung oder Verringerung der Schwere oraler Mucositis in einem Individuum das eine Krebstherapie erhält..

2. Verbindung zur Verwendung nach Anspruch 1, worin die Verbindung der Formel I das (+)-Trans-Isomer ist, dargestellt durch die Formel 1A: 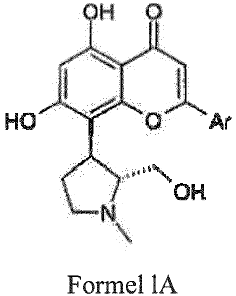 worin Ar Phenyl ist, das substituiert ist mit 1, 2, oder 3 identischen oder verschiedenen Substituenten ausgewählt unter Chlor, Brom, Fluor und Iod; C₁-C₄-Alkyl und Trifluormethyl, oder ein pharmazeutisch annehmbares Salz oder ein Solvat davon.

3. Verbindung zur Verwendung nach Anspruch 1 oder Anspruch 2, worin die Verbindung dem Individuum vor der Krebstherapie, gleichzeitig mit der Krebstherapie, nach der Krebstherapie oder zwischen Krebstherapien verabreicht wird.

4. Verbindung zur Verwendung nach Anspruch 3, worin die Verbindung dem Individuum vor der Krebstherapie verabreicht wird.

5. Verbindung zur Verwendung nach Anspruch 3, worin die Verbindung dem Individuum gleichzeitig mit der Krebstherapie verabreicht wird.

6. Verbindung zur Verwendung nach Anspruch 3, worin die Verbindung dem Individuum nach der Krebstherapie verabreicht wird.

7. Verbindung zur Verwendung nach Anspruch 3, worin die Verbindung dem Individuum zwischen Krebstherapien verabreicht wird.

8. Verbindung zur Verwendung gemäß einem der vorstehenden Ansprüche 1 bis 7, worin das Individuum, das eine Krebstherapie erhält, ein Patient ist, der an akuter myeloider Leukämie, akuter lymphoider Leukämie, chronischer myeloider Leukämie, Hodgkins Erkrankung, multiplem Myelom, nicht-Hodgkins Erkrankung oder Kopf- und Nacken-Krebs leidet.

9. Verbindung der Formel (I) zur Verwendung nach Anspruch 8, worin das Individuum, das eine Krebstherapie erhält ein Patient ist, der an Kopf- und Nacken-Krebs leidet.

10. Verbindung der Formel (I) zur Verwendung nach eine der Ansprüche 1 bis 9, worin die Verbindung (+)-trans-2-(2-Chlorphenyl)-5,7-dihydroxy-8-(2-hydroxymethyl-1-methylpyrrolidin-3-yl)-chromen-4-on-hydrochloride (Verbindung A) oder (+)-trans-2-(2-Chlor-4-trifluormethyl-phenyl)-5,7-dihydroxy-8-(2-hydroxymethyl-1-methylpyrrolidin-3-yl)-chromen-4-on-hydrochlorid (Verbindung B) ist.

11. Verbindung zur Verwendung nach Anspruch 10, worin die Verbindung A in einer Dosis von 9 mg/m²/Tag bis 259 mg/m²/Tag verabreicht werden soll.

12. Verbindung zur Verwendung nach einem der vorstehenden Ansprüche 1 bis 11, worin die Krebstherapie Strahlungstherapie, Chemotherapie oder eine Kombination davon ist.

13. Verbindung zur Verwendung nach Anspruch 12, worin die kumulative Strahlendosis, die einem bedürftigen Individuum verabreicht werden soll, ausgewählt ist unter 30 Grays bis 82 Grays.

14. Verbindung zur Verwendung nach Anspruch 13, worin die Chemotherapie die Verwendung von Cisplatin beinhaltet, wahlweise worin die Dosis an einem bedürftigen Individuum zu verabreichendem Cisplatin von 30 mg/m² bis 40 mg/m² beträgt.

15. Pharmazeutische Zusammensetzung zur Verwendung bei der Verhinderung, Behandlung oder Reduktion der Schwere oraler Mucositis in einem Individuum, das einer Krebstherapie erhält, welche eine therapeutisch wirksame Menge der Verbindung der Formel (I) umfasst. 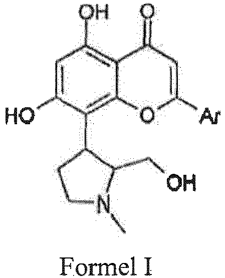 worin Ar Phenyl ist, das substituiert ist mit 1 oder 2 identischen oder verschiedenen Substituenten ausgewählt unter Chlor, Brom, Fluor und Iod, C₁-C₄-Alkyl und Trifluormethyl, oder ein pharmazeutisch annehmbares Salz, ein Solvat, ein Stereoisomer oder ein Diastereoisomer davon, und einen pharmazeutisch annehmbaren Träger.

## Revendications

1. Composé de formule (I), 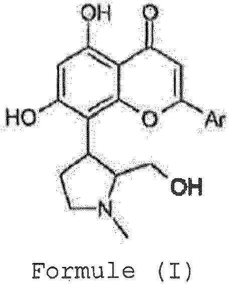 dans laquelle Ar est un groupe phényle substitué avec 1, 2 ou 3 substituants identiques ou différents choisis parmi le chlore, le brome, le fluor, l'iode, un alkyle en C₁ à C₄ et le trifluorométhyle ; ou un sel, solvate, stéréoisomère ou diastéréoisomère pharmaceutiquement acceptable de celui-ci, pour son utilisation dans la prévention, le traitement ou la réduction de la gravité d'une mucosite orale chez un sujet soumis à un traitement anticancéreux.

2. Composé pour l'utilisation selon la revendication 1, dans lequel le composé de formule (I) est un isomère (+)-trans représenté par la formule (IA), 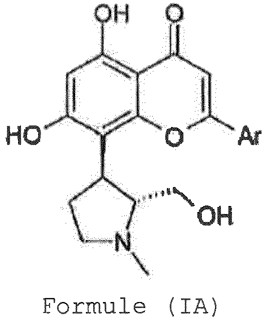 dans laquelle Ar est un groupe phényle, qui est substitué avec 1, 2 ou 3 substituants identiques ou différents choisis parmi le chlore, le brome, le fluor, l'iode, un alkyle en C₁ à C₄ et le trifluorométhyle ; ou un sel ou solvate pharmaceutiquement acceptable de celui-ci.

3. Composé pour l'utilisation selon la revendication 1 ou la revendication 2, dans lequel le composé est administré au sujet, avant un traitement anticancéreux, en même temps que le traitement anticancéreux, après le traitement anticancéreux ou entre deux traitements anticancéreux.

4. Composé pour l'utilisation selon la revendication 3, dans lequel le composé doit être administré au sujet avant le traitement anticancéreux.

5. Composé pour l'utilisation selon la revendication 3, dans lequel le composé doit être administré au sujet en même temps que le traitement anticancéreux.

6. Composé pour l'utilisation selon la revendication 3, dans lequel le composé doit être administré au sujet après le traitement anticancéreux.

7. Composé pour l'utilisation selon la revendication 3, dans lequel le composé doit être administré au sujet entre deux traitements anticancéreux.

8. Composé pour l'utilisation selon l'une quelconque des revendications 1 à 7 précédentes, dans lequel le sujet soumis au traitement anticancéreux est un patient souffrant d'une leucémie myéloïde aiguë, d'une leucémie lymphoïde aiguë, d'une leucémie myéloïde chronique, de la maladie de Hodgkin, d'un myélome multiple, d'une maladie non hodgkinienne ou d'un cancer de la tête et du cou.

9. Composé de formule (I) pour l'utilisation selon la revendication 8, dans lequel le sujet soumis au traitement anticancéreux est un patient souffrant d'un cancer de la tête et du cou.

10. Composé pour l'utilisation selon l'une quelconque des revendications 1 à 9 précédentes, dans lequel le composé est le chlorhydrate de (+)*-trans-*2-(2-chorophényl)-5,7-dihydroxy-8-(2-hydroxyméthyl-1-méthylpyrrolidin-3-yl)-chromèn-4-one (composé A) ou le chlorhydrate de (+)-*trans*-2-(2-choro-4-trifluoro-méthyl-phényl)-5,7-dihydroxy-8-(2-hydroxyméthyl-1-méthylpyrrolidin-3-yl)-chromèn-4-one (composé B).

11. Composé pour l'utilisation selon la revendication 10, dans lequel le composé A doit être administré à une dose de 9 mg/m²/jour à 259 mg/m²/jour.

12. Composé pour l'utilisation selon l'une quelconque des revendications 1 à 11 précédentes, dans lequel le traitement anticancéreux est une radiothérapie, une chimiothérapie ou leur combinaison.

13. Composé pour l'utilisation selon la revendication 12, dans lequel la dose cumulée du rayonnement à administrer à un sujet en ayant besoin est choisie parmi 30 Grays à 82 Grays.

14. Composé pour l'utilisation selon la revendication 13, dans lequel la chimiothérapie implique l'utilisation de cisplatine, facultativement dans lequel la dose de cisplatine à administrer à un sujet en ayant besoin est de 30 mg/m² à 40 mg/m².

15. Composition pharmaceutique pour son utilisation dans la prévention, le traitement ou la réduction de la gravité d'une mucosite orale chez un sujet soumis à un traitement anticancéreux, comprenant une quantité thérapeutiquement efficace d'un composé de formule (I), 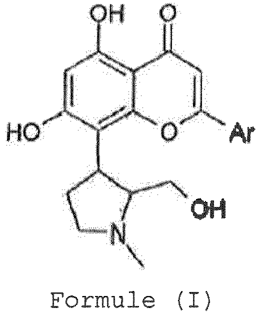 dans laquelle Ar est un groupe phényle substitué avec 1 ou 2 substituants identiques ou différents choisis parmi le chlore, le brome, le fluor, l'iode, un alkyle en C₁ à C₄ et le trifluorométhyle ; ou un sel, solvate, stéréoisomère ou diastéréoisomère pharmaceutiquement acceptable de celui-ci et un support pharmaceutiquement acceptable.
